# EUROPEAN PATENT SPECIFICATION

(11) **EP 0 821 955 B1**
(45) Date of publication and mention of the grant of the patent: **20.02.2002**
(21) Application number: 97305585.8
(22) Date of filing: 25.07.1997
(51) Int. Cl.: A61K 31/4439, A61P 25/00

(54) **Use of 3-(4-hexyloxy-1,2,5-thiadiazol-3-yl)-1,2,5,6-tetrahydro-1-methylpyridine (xanomeline) for treating bipolar disorder**
Verwendung von 3-(4-Hexyloxy-1,2,5-Thiadiazol-3-yl)-1,2,5,6-Tetrahydro-1-Methylpyridin (Xanomelin) zur Behandlung von bipolaren Störungen
Utilisation de la 3-(4-hexyloxy-1,2,5-thiadiazol-3-yl)-1,2,5,6-tétrahydro-1-méthylpyridine (xanoméline) pour traiter les troubles bipolaires

(30) Priority: 01.08.1996 US 22900 P
(43) Date of publication of application: 04.02.1998
(73) Proprietor: ELI LILLY AND COMPANY, Indianapolis, Indiana 46285 (US)
(72) Inventor: Bymaster, Franklin Porter, Brownsburg, Indiana 46112 (US); Shannon, Harlan Edgar, Carmel, Indiana 46033 (US)
(74) Representative: Pritchard, Judith

(56) References cited:
- EP-A- 0 384 288
- EP-A- 0 709 094
- EP-A- 0 709 095
- EP-A- 0 709 381
- WO-A-94/20495
- WO-A-95/05174
- SAUERBERG P ET AL: "NOVEL FUNCTIONAL M1 SELECTIVE MUSCARINIC AGONISTS. SYNTHESIS AND STRUCTURE-ACTIVITY RELATIONSHIPS OF 3-(1,2,5-THIADIAZOLYL)-1,2,5,6- TETRAHYDRO-1-METHYLPYRIDINES" JOURNAL OF MEDICINAL CHEMISTRY, vol. 35, no. 12, 12 June 1992, pages 2274-2283, XP000562652

## Description

This invention provides the use for the manufacture of a medicament for treating or alleviating the symptoms of bipolar disorder of 3-(4-hexyloxy-1,2,5-thiadiazol-3-yl)-1,2,5,6-tetrahydro-1-methylpyridine (hereinafter referred to as "xanomeline").

Bipolar Disorder is a psychiatric condition which is prevelant across cultures and age groups. The lifetime prevalence of Bipolar Disorder can be as high as 1.6%. DSM-IV, p. 353 (American Psychiatric Association, Washington, D.C. 1994). Bipolar Disorder is a recurrent disorder characterized by one or more Manic Episodes immediately before or after a Major Depressive Episode or may be characterized by one or more Major Depressive Episodes accompanied by at least one Hypomanic Episode.
Additionally, the symptoms must cause clinically significant distress or impairment in social, occupational, or other important areas of functioning. In some cases the Hypomanic Episodes themselves do not cause impairment; however, the impairment may result from the Major Depressive Episodes or from a chronic pattern of unpredictable mood episodes and fluctuating unreliable interpersonal and occupational functioning. The symptoms of Bipolar Disorder must not be better accounted for by a psychotic condition or due to the direct physiological effects of a medication, other somatic treatments for depression, drugs of abuse, or toxin exposure.

Bipolar Disorder is associated with a significant risk of completed suicide. Further, the patient suffering from Bipolar Disorder is likely to suffer from school truancy, school failure, occupational failure, or divorce.

Therefore, Bipolar Disorder is a serious, fairly prevelant, psychological condition which is clearly distinguished from psychotic conditions such as schizophrenia. DSM-IV, p. 353 (American Psychiatric Association, Washington, D.C. 1994). DSM-IV, p. 353 (American Psychiatric Association, Washington, D.C. 1994).

Applicants have discovered that xanomeline, thought to be a muscarinic agonist, can be useful for treating Bipolar Disorder. The present invention relates to a medicament for treating Bipolar Disorder. More specifically, the invention provides a medicament for treating Bipolar Disorder in humans using xanomeline.

As noted hereinbefore, the compound employed in the use of the present invention is known. Methods of preparing the compound, as well as pharmaceutical formulations containing the compound, are taught by Sauerberg in U.S. Pat. No. 5,043,345 (hereinafter refered to as the "'345 patent").
The '345 patent teaches that xanomeline can be useful for treating Alzheimer's Disease and as stimulants of the cognitive function of the forebrain and hippocampus of mammals. Applicants have discovered that xanomeline can be useful for the treatment of bipolar disorder. Xanomeline may address the long felt need for treatments having an acceptable safety profile and provide effective relief to the patient suffering from bipolar disorder.
EP 709381 discloses azacyclic or azabicyclic compounds useful in treating diseases in the central nervous system caused by malfunctioning of the muscarinic cholinergic system.
EP 709094 discloses a method for treating anxiety using a tetrahydropyridine oxadiazole or thiadiazole compound.
EP 709095 relates to a method for treating anxiety using a tetrahydropyridine or azabicyclic oxadiazole or thiadiazole compound.

The present invention provides a use of a compound of Formula I: 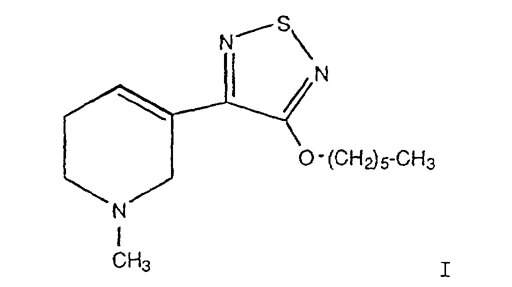 or
a pharmaceutically acceptable salt or solvate thereof for the manufacture of a medicament for treating Bipolar Disorder.

The term "effective amount", as used herein, represents an amount of compound necessary to prevent or treat a human susceptible to or suffering from Bipolar Disorder following administration to such human. The active compound is effective over a wide dosage range. For example, dosages per day will normally fall within the range of 0.005 to 500 mg/kg of body weight. In the treatment of adult humans, the range of 0.05 to 100 mg/kg, in single or divided doses, is preferred. A particularly preferred range is from 1 mg/kg to 100 mg/kg per day, more particularly from 10 mg/kg to 100 mg/kg per day. However, it will be understood that the amount of the compound actually administered will be determined by a physician, in the light of the relevant circumstances including the condition to be treated, the choice of compound to be administered, the age, weight, and response of the individual patient, the severity of the patient's symptoms, and the chosen route of administration. While the present compound may be administered orally to humans susceptible to or suffering from Bipolar Disorder, the compound is particularly well suited to be administered transdermally. When the compound is delivered transdermally, it is preferred that the effective amount is from 10 mg to 100 mg per day delivery of base compound. It is especially preferred that such patch delivers an effective amount for one to seven days.

The compound may further be delivered by a variety of other pharmaceutically accepted routes including, parenterally, subcutaneous, intranasal, intramuscular and intravenous routes. Such formulations may be designed to provide delayed or controlled release using formulation techniques which are known in the art.

As used herein the term "treating" includes prophylaxis of a physical and/or mental condition or amelioration or elimination of the developed physical and/or mental condition once it has been established or alleviation of the characteristic symptoms of such condition.

As used herein, the term "Bipolar Disorder" shall refer to a condition characterized as a Bipolar Disorder, in the DSM-IV-R. Diagnostic and Statistical Manual of Mental Disorders, Revised, 3rd Ed. (1994) as catagory 296.xx. To further clarify, Applicants contemplate the treatment of both Bipolar Disorder I and Bipolar disorder II as described in the DSM-IV-R. The DSM-IV-R was prepared by the Task Force on Nomenclature and Statistics of the American Psychiatric Association, and provides clear descriptions of diagnostic catagories. The skilled artisan will recognize that there are alternative nomenclatures, nosologies, and classification systems for pathologic psychological conditions and that these systems evolve with medical scientific progress.

The compounds employed in the invention are not believed to act via the GABA/benzodiazepine, serotonin, or dopamine receptor systems in humans. Rather, the activity of the present compound as a treatment for Bipolar Disorder is believed to be based upon modulation of muscarinic cholinergic receptors. However, the mechanism by which the present compounds function is not necessarily the mechanism stated *supra.*

Xanomeline has been studied using accepted pharmacological methods such as oxotremorine-M verses N-methylscopolamine binding studies (Freedman et al. Br. J. Pharmacology, **93**:437-445 (1988). Xanomeline inhibited the binding of ³H-oxotremorine-M with an inhibition constant (Kᵢ) of 2nM. The binding of the muscarinic m1 antagonist ligand, ³H-pirenzepine, to m1 receptors in hippocampus and ³H-quinuclidinyl benzilate to m2 receptors in brain stem was inhibited with Kᵢ values of 5 and 24 nM, respectively.

Muscarinic agonists stimulate the formation of cAMP up to 10 fold in Chinese Hamster Ovary (CHO) m4 cells treated with pertussisi toxin and the pharmacology is consistent with the mediation by m4 receptors. Eckols K. Soc. Neurosci Abstr., **21**:2040 (1995). In this assay, xanomeline efficaciously and potently stimulated the formation of cAMP. Such studies suggest that xanomeline predominantly activates m1 and m4 receptors.

Xanomeline can be prepared as described in the '345 patent.

The following Examples are studies to establish the usefulness of the named compounds for treating Bipolar Disorder.

### Example 1

### Human Clinical Trials

The activity of xanomeline for treating or alleviating Bipolar Disorder can be demonstrated by human clinical trials. The study was designed as a double-blind, parallel, placebo-controlled multicenter trial. The subjects were randomized into four groups, placebo and 25, 50, and 75 mg tid of test compound. The dosages were administered orally with food. Subjects were observed at four visits to provide baseline measurements. Visits 5-33 served as the treatment phase for the study.

During the visits, subjects are observed for signs of agitation, mood swings, tremor, delirium, social withdrawal, and concentration abilities.

Treatment groups are compared with respect to the number and percent of subjects who ever had the symptom during the double-blind portion of the study (visits 5 through 33), at a severity that was worse than during the baseline visits (1 through 4).

## Claims

1. A use of a compound of Formula I: 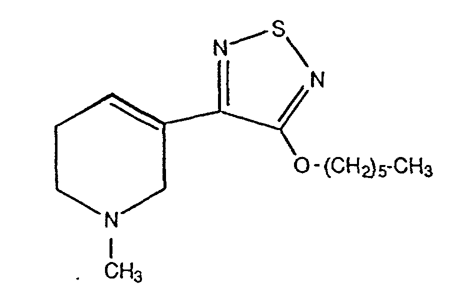 or a pharmaceutically acceptable salt thereof for the manufacture of a medicament for treating Bipolar Disorder.

2. A use of Claim 1 wherein the amount of compound of Formula I to be administered is from 1 mg/kg to 100 mg/kg per day.

3. A use of Claim 2 wherein the amount of compound of Formula I to be administered is from 10 mg/kg to 100 mg/kg per day.

4. A use of Claim 1 wherein the medicament is in the form of a transdermal patch.

5. A use of Claim 4 wherein the transdermal patch is to deliver from 10 to 100 mg of base compound per day.

6. A use of Claim 5 wherein the transdermal patch is to deliver an effective amount for one (1) to seven (7) days.

7. A use of Claim 1 wherein the Bipolar Disorder is Bipolar Disorder I.

8. A use of Claim 1 wherein the Bipolar Disorder is Bipolar Disorder II.

## Patentansprüche

1. Verwendung einer Verbindung der Formel I 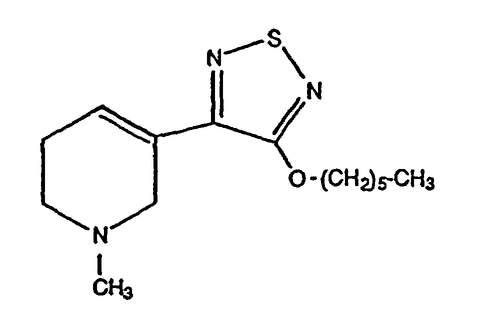 oder eines pharmazeutisch annehmbaren Salzes hiervon zur Herstellung eines Arzneimittels zur Behandlung der bipolaren Störung.

2. Verwendung nach Anspruch 1, worin die Menge der zu verabreichenden Verbindung der Formel I 1 mg/kg bis 100 mg/kg pro Tag beträgt.

3. Verwendung nach Anspruch 2, worin die Menge der zu verabreichenden Verbindung der Formel I 10 mg/kg bis 100 mg/kg pro Tag beträgt.

4. Verwendung nach Anspruch 1, worin das Arzneimittel in Form eines Transdermalpflasters vorliegt.

5. Verwendung nach Anspruch 4, worin das Transdermalpflaster 10 bis 100 mg der Basisverbindung pro Tag verabreichen soll.

6. Verwendung nach Anspruch 5, worin das Transdermalpflaster eine wirksame Menge für ein (1) bis sieben (7) Tage verabreichen soll.

7. Verwendung nach Anspruch 1, worin die bipolare Störung die bipolare Störung I ist.

8. Verwendung nach Anspruch 1, worin die bipolare Störung die bipolare Störung II ist.

## Revendications

1. Utilisation d'un composé de formule I : 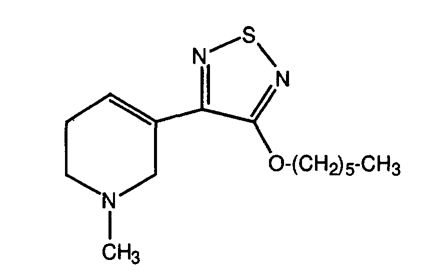 ou d'un de ses sels pharmaceutiquement acceptables pour la fabrication d'un médicament destiné au traitement du trouble bipolaire.

2. Utilisation de la revendication 1 dans laquelle la quantité de composé de formule I à administrer est de 1 mg/kg à 100 mg/kg par jour.

3. Utilisation de la revendication 2 dans laquelle la quantité de composé de formule I à administrer est de 10 mg/kg à 100 mg/kg par jour.

4. Utilisation de la revendication 1 dans laquelle le médicament est sous la forme d'un timbre transdermique.

5. Utilisation de la revendication 4 dans laquelle le timbre transdermique doit délivrer de 10 à 100 mg de composé de base par jour.

6. Utilisation de la revendication 5 dans laquelle le timbre transdermique doit délivrer une quantité efficace pendant un (1) à sept (7) jours.

7. Utilisation de la revendication 1 dans laquelle le trouble bipolaire est le trouble bipolaire I.

8. Utilisation de la revendication 1 dans laquelle le trouble bipolaire est le trouble bipolaire II.
